(19) Europäisches Patentamt
European Patent Office
Office européen des brevets

(11) Veröffentlichungsnummer: **0 325 263 B1**

(12) **EUROPÄISCHE PATENTSCHRIFT**

(45) Veröffentlichungstag der Patentschrift: **23.09.92**

(51) Int. Cl.$^5$: **C07D 211/70**, A61K 31/44

(21) Anmeldenummer: **89100918.5**

(22) Anmeldetag: **20.01.89**

Die Akte enthält technische Angaben, die nach dem Eingang der Anmeldung eingereicht wurden und die nicht in dieser Patentschrift enthalten sind.

(54) **Tetrahydropyridin-Derivate.**

(30) Priorität: **21.01.88 DE 3801659**

(43) Veröffentlichungstag der Anmeldung:
**26.07.89 Patentblatt 89/30**

(45) Bekanntmachung des Hinweises auf die Patenterteilung:
**23.09.92 Patentblatt 92/39**

(84) Benannte Vertragsstaaten:
**AT BE CH DE ES FR GB GR IT LI LU NL SE**

(56) Entgegenhaltungen:
**DE-A- 1 929 921**
**US-A- 4 467 095**

**Hansch et al.: "Comprehensive Medicinal Chemistry" vol. 3, pp 456-466**

(73) Patentinhaber: **BOEHRINGER INGELHEIM KG**
**Postfach 200**
**W-6507 Ingelheim am Rhein(DE)**
(84) Benannte Vertragsstaaten:
**AT BE CH DE ES FR GB GR IT LI LU NL SE**

(73) Patentinhaber: **BOEHRINGER INGELHEIM INTERNATIONAL GmbH**
**Postfach 20**
**W-6507 Ingelheim am Rhein(DE)**
(84) Benannte Vertragsstaaten:
**GB**

(72) Erfinder: **Walther, Gerhard, Dr.**
**Pfarrer-Heberer-Strasse 37**
**W-6530 Bingen(DE)**
Erfinder: **Weber, Karl-Heinz, Dr.**
**Kaiser-Karl-Strasse 11**
**W-6535 Gau-Algesheim(DE)**
Erfinder: **Stransky, Werner, Dr.**
**Im Hippel 24**
**W-6535 Gau-Algesheim(DE)**
Erfinder: **Kuhn, Franz, Dr.**
**Beethovenstrasse 11**
**W-6535 Gau-Algesheim(DE)**
Erfinder: **Lehr, Erich, Dr.**
**In der Toffel 5**
**W-6531 Waldalgesheim(DE)**
Erfinder: **Müller, Enzio, Prof. Dr.**
**Grundstrasse 22**
**W-6507 Ingelheim am Rhein(DE)**
Erfinder: **Schingnitz, Günter, Dr.**
**Unter den Gärten 10**
**W-6550 Bad Kreuznach(DE)**

**EP 0 325 263 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Ensinger, Helmut, Dr.**
**Magdeburger Strasse 54**
**W-6507 Ingelheim am Rhein(DE)**

## Beschreibung

Die Erfindung betrifft neue Tetrahydropyridin-Derivate, Verfahren zur Herstellung und ihre Verwendung als Arzneimittel.

Die neuen Verbindungen entsprechen der allgemeinen Formel

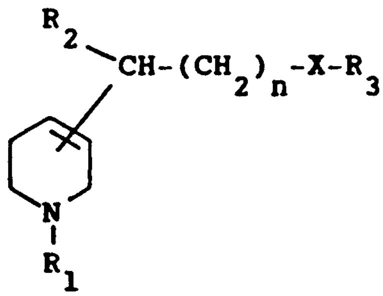

I

worin

- $R_1$ Wasserstoff, eine verzweigte oder unverzweigte Alkylgruppe mit 1 bis 3 Kohlenstoffatomen;
- $R_2$ Wasserstoff oder eine Methylgruppe;
- $R_3$ eine verzweigte oder unverzweigte Alkylgruppe mit 1 bis 4 Kohlenstoffatomen, eine Allylgruppe oder eine Propargylgruppe;
- X Sauerstoff oder Schwefel; und
- n eine der Zahlen 0 oder 1 bedeuten, gegebenenfalls als Racemate oder als optisch aktive Verbindungen sowie gegebenenfalls deren Säureadditionssalze wie auch deren quartäre Verbindungen.

Bevorzugt sind Verbindungen der allgemeinen Formel I worin $R_1$ Methyl, $R_2$ Wasserstoff, $R_3$ Methyl, Ethyl oder n-Propyl und $n = 0$ bedeuten.

Weiterhin bevorzugt sind Verbindungen der allgemeinen Formel I worin die 4-Position substituiert ist.

Bevorzugte quartäre Verbindungen der allgemeinen Formel I

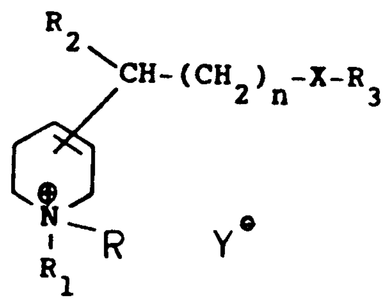

enthalten R in der Bedeutung von Methyl oder Ethyl, wobei $y^-$ das entsprechende Anion, bevorzugt ein Halogen ist.

Als Beispiele für Alkylgruppen mit 1 bis 4 Kohlenstoffatomen werden unter anderem Methyl, Ethyl, n-Propyl, Isopropyl, n-Butyl, sec.-Butyl und iso-Butyl verstanden. Halogen bedeutet Fluor, Chlor, Brom oder Jod.

Das 4-(Methoxyethyl)-1-methyl-1,2,3,6-tetrahydropyridin ist bekannt (CA 68 P 105016s), seine Verwendung als Arzneimittel jedoch bislang nicht beschrieben.

Die Verbindungen der allgemeinen Formel I besitzen wertvolle pharmakologische Eigenschaften.

So zeigen sie in Bindungsstudien Affinitäten zu muskarinen Rezeptoren und muskarin-agonistische GTP-shifts (GTP = Guanosintriphosphat). (Birdsall, N.I.M., E.C. Hulme and J.M. Stockton 1984 in T.I.P.S. Supplement, Proc. Internat. Symposium on Subtypes of Muscarinic Receptors, Ed. Hirschowitz, Hammer, Giacchetti, Keirns, Levine; Elsevier p. 4-8.).

Die Rezeptorbindungsstudien wurden gemäß dem nachstehenden Literaturzitat ausgeführt.

A. Closse, H. Bittiger, D. Langenegger und A. Wanner, Naunyn-Schmiedeberg's Arch. Pharmacol. 335, 372

- 377 (1987).
Die Ergebnisse sind in Tabelle A dargestellt.

## Tabelle A: Rezeptorbindungsstudien

**Radioligand:** L(+)cis-[2-Methyl-$^{3}$H]-N,N,N-trimethyl - 1,3-dioxolan-4-methanammonium-jodid NET-647, Fa. NEN (New England Nuclear; DU PONT).

**Organ:** Cerebraler Cortex (Ratte)

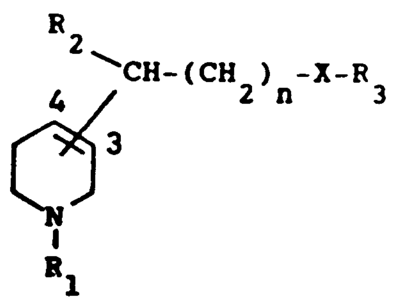

I

| Beispiel | $R_1$ | $R_2$ | $R_3$ | n | Subst. Pos. | X | Ki [nmol/l] |
|---|---|---|---|---|---|---|---|
| 1 MA | $-CH_3$ | -H | $-CH_3$ | 0 | 4 | -O- | 337 |
| 2 MA | $-CH_3$ | -H | $-C_2H_5$ | 0 | 4 | -O- | 210 |
| 9 MA | $-CH_3$ | -H | $-n-C_3H_7$ | 0 | 4 | -O- | 140 |
| 13 MA | $-CH_3$ | -H | $-CH_3$ | 0 | 4 | -S- | 15 |
| 4 MA | $-CH_3$ | -H | $-C_2H_5$ | 0 | 4 | -S- | 8,3 |
| 19 OX | $-CH_3$ | -H | $-C_2H_5$ | 0 | 3 | -S- | 690 |
| OX | $-CH_3$ | -H | $-CH_3$ | 1 | 4 | -O- | 3900 |

MA = Maleinat, OX = Oxalat

In pharmakologischen Testmodellen wurde in vitro und in vivo eine cholinerge Wirkung nachgewiesen. So zeigen beispielsweise 4-Methoxymethyl-1-methyl-1, 2, 3, 6-tetrahydro-pyridin und 4-Ethoxymethyl-1-methyl-1,2,3,6-tetrahydro-pyridin in einer Dosis von 1 mg/kg und 3 mg/kg i.v. sowie 1-Methyl-4-methylthiomethyl-1,2,3,6-tetrahydro-pyridin in einer Dosis von 0,1 mg/kg und 0.3 mg/kg i.v. im EEG (Elektroenzephalogramm) des wachen Kaninchens eine für Cholinomimetika typische Arousal-Reaktion.

Als muskarine Agonisten (Cholinomimetika) sind die Substanzen zur Therapie von Krankheiten bei einer Unterfunktion des cholinergen Systems geeignet.

Aufgrund pharmakologischer Befunde sind die Verbindungen z.B. zur Behandlung nachfolgend genannter Krankheiten geeignet: Morbus Alzheimer, senile Demenz, kognitive Störungen und außerdem können die Verbindungen zur Verbesserung der Gedächtnisleistung eingesetzt werden.

Quartäre Verbindungen der allgemeinen Formel I eignen sich besonders zur peripheren Anwendung, so z.B. zur Glaukombehandlung.

Die Verbindungen der allgemeinen Formel I können nach bekannten Analogieverfahren hergestellt werden.

Verbindungen der Formel I mit $R_1$ ungleich Wasserstoff lassen sich gemäß dem nachfolgenden Reaktionsschema I synthetisieren.

Reaktionsschema 1:

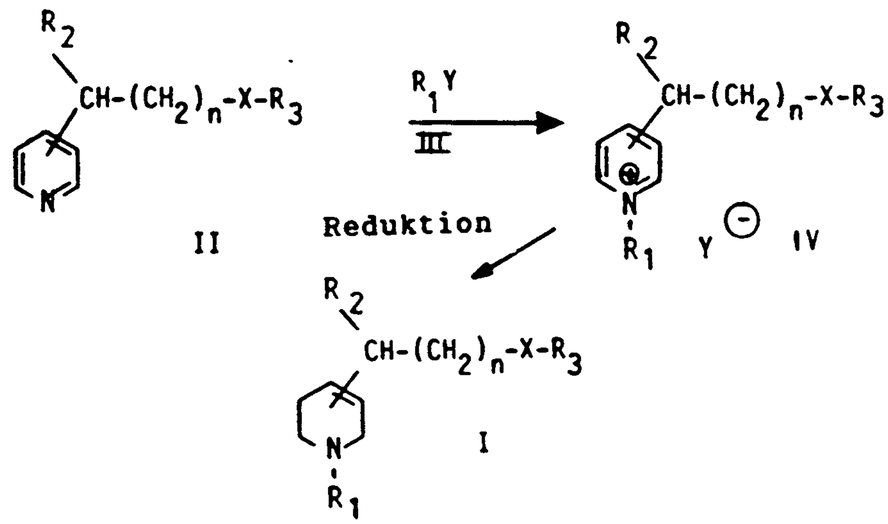


Ausgehend von bekannten - oder aber nach Analogieverfahren herstellbaren - Pyridinderivaten der allgemeinen Formel II, worin n, X, $R_2$ und $R_3$ die vorgenannte Bedeutung aufweisen, erfolgt eine N-Alkylierung durch ein Alkylierungsmittel der Formel $R_1Y$ - worin $R_1$ die vorgenannte Bedeutung aufweist und Y ein geeignetes Anion ist - beispielsweise durch Umsetzung mit den entsprechenden

a) Alkylhalogeniden, besonders Alkylbromiden oder Alkyljodiden

b) Alkyl- oder Arylsulfonsäureestern, besonders Methan- oder p-Toluolsulfonsäureestern

c) Schwefelsäureestern, z. B. Dimethylsulfat

in Aceton, Acetonitril, Toluol, Ethanol u.a. bei Temperaturen zwischen der Raumtemperatur und dem Siedepunkt der Reaktionsmischung.

Die Reduktion der Quartärverbindungen IV zu den Vebindungen der allgemeinen Formel I erfolgt ebenfalls nach bekannten Verfahren, so z.B. Methoden mit komplexen Metallhydriden (z.B. Andor Hajos, Komplexe Hydride, VEB Deutscher Verlag der Wissenschaften, Berlin 1966, S. 208 bzw. S. 431), wie z.B. Natriumborhydrid, in geeigneten Lösungsmitteln; bevorzugt wird die Reduktion mit Natriumborhydrid in Alkoholen, wie Methanol oder Ethanol bei 0 - 25 °C durchgeführt.

Verbindungen der Formel I mit $R_1$ = H erhält man durch Entmethylierung von Verbindungen der Formel I ($R_1$ = $-CH_3$),z.B. durch Umsetzung mit Phosgen in Toluol und anschliessender Hydrolyse der intermedinär entstehenden N-Chlorcarbonyl-Verbindungen (R. Banholzer et al, Arzneimittelforschung 35 (I), 217-228 (1985)).

Die Quartärverbindungen der allgemeinen Formel I können analog den Quartärsalzen IV durch Umsetzung von Verbindungen der Formel I mit Alkylierungsreagenzien dargestellt werden.

Die Verbindungen der allgemeinen Formel I können allein oder in Kombination mit anderen erfindungsgemäßen Wirkstoffen, gegebenenfalls auch in Kombination mit weiteren pharmakologisch aktiven Wirkstoffen,z.B. weiteren Cerebroaktivatoren zur Anwendung gelangen. Geeignete Anwendungsformen sind beispielsweise Tabletten, Kapseln, Zäpfchen, Lösungen, Säfte, Emulsionen oder dispersible Pulver.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphtalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowei ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Die therapeutisch wirksame Einzeldosis liegt im Bereich zwischen 1 und 100 mg.

Die nachfolgenden Beispiele illustrieren die vorliegende Erfindung ohne sie jedoch in ihrem Umfang zu beschränken:

Pharmazeutische Formulierungsbeispiele

| A) Tabletten | pro Tablette |
|---|---|
| Wirkstoff | 80 mg |
| Milchzucker | 140 mg |
| Maisstärke | 240 mg |
| Polyvinylpyrrolidon | 15 mg |
| Magnesiumstearat | 5 mg |
| | 480 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet , knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) Tabletten | pro Tablette |
|---|---|
| Wirkstoff | 60 mg |
| Maisstärke | 190 mg |
| Milchzucker | 55 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 380 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke

und Wasser zu einem Granulat verarbeitet, welches getrocknet und gesiebt wird. Dazu gibt man die Natrium-carboxy-methylstärke und das Magnesiumstearat, vermischt und verpreßt das Gemisch zu Tabletten geeigneter Größe.

| C) Ampullen | |
|---|---|
| Wirkstoff | 50 mg |
| Natriumchlorid | 10 mg |
| bidestilliertes Wasser q.s. ad | 1,0 ml |

Herstellung:

Der Wirkstoff und das Natriumchlorid werden in bidestilliertem Wasser gelöst und die Lösung in Ampullen steril abgefüllt.

| D) Tropfen | |
|---|---|
| Wirkstoff | 5,0 g |
| p-Hydroxybenzoesäuremethylester | 0,1 g |
| p-Hydroxybenzoesäurepropylester | 0,1 g |
| entmineralisiertes Wasser q.s. ad | 100,0 ml |

Herstellung:

Der Wirkstoff und die Konservierungsmittel werden in demineralisiertem Wasser gelöst und die Lösung filtriert und in Flaschen zu je 100 ml abgefüllt.

Beispiel 1:

4 Methoxymethyl-1-methyl-1,2,3,6-tetrahydro-pyridin

a) 4-Methoxymethyl-1-methyl-pyridinium-iodid

Eine Lösung von 4,8 g (0,039 Mol) 4-Methoxymethylpyridin in 25 ml Acetonitril wird mit 6,6 g (0,047 Mol) Methyljodid versetzt und fünf Stunden bei Raumtemperatur gerührt. Anschließend fällt man das teilweise ausgefallene Quartärsalz durch Zugabe von Essigester aus. Man erhält 9,1 g (88 % d. Therorie) 4-Methoxymethyl-1-methyl-pyridiniumjodid vom Schmelzpunkt 105-108 ° C.

Das analysenreine Methojodid hat einen Schmelzpunkt von 106-108 ° C ($CH_3CN$).

| $[C_8H_{12}NO]^+ J^-$ (265,10) | | | | |
|---|---|---|---|---|
| ber. | C 36.25 | H 4,57 | H 5,28 | J 47,87 |
| gef. | 36.02 | 4,60 | 5,26 | 47,69 |

b) In eine Lösung von 38,4 g (0,145 Mol) 4-Methoxymethyl-1-methyl-pyridinium-jodid in 380 ml Methanol werden unter Rühren bei 10-15 ° C 13,15 g (0,348 Mol)Natriumborhydrid portionsweise eingetragen. Die Reaktionsmischung wird anschließend noch zwei Stunden bei Raumtemperatur nachgerührt, mit 2N Salzsäure angesäuert und im Vakuum eingedampft. Der Rückstand wird mit 40 %iger wässeriger Kaliumkarbonat-Lösung versetzt und mit Ether extrahiert. Das beim Einengen der über wasserfreiem Natriumsulfat getrockneten organischen Phase zurückbleibende Öl wird destilliert.

Ausbeute: 17,6 g, farbloses Öl (86 % d. Theorie); Kp. 70-72 ° C/20 mbar.

$^1$H NMR ($CDCl_3$; 90 MHz)

$\delta$ 5.65 (m,1H,CH=); 3.85(m,2H,O-$CH_2$); 3.32 (s,3H,$OCH_3$); 2.95 (m,2H,$NCH_2$); 2.50(m,2H,$NCH_2$); 2.33-(s,3H,$NCH_3$); 2.18(m,2H,$CH_2$-C=).

Das entsprechende 4-Methoxymethyl-1-methyl-1,2,3,6-tetrahydro-pyridin-maleinat (1:1) hat einen Schmelzpunkt von 86-87 ° C (Essigester).

Beispiel 2:

4-Ethoxymethyl-1-methyl-1,2,3,6-tetrahydropyridin

Eine Lösung von 5,9 g (0,043 Mol) 4-Ethoxymethylpyridin und 7,33 g (0,052 Mol) Methyljodid in 50 ml Acetonitril wird 14 Stunden bei Raumtemperatur gerührt. Anschließend wird die Reaktionslösung im Vakuum eingedampft. Der Rückstand (12,1 g) wird in 120 ml Methanol gelöst und analog Beispiel 1 b) mit 3,94 g Natriumborhydrid reduziert.

Ausbeute: 4,7 g (70 % d. Theorie); Kp. 82-83 ° C/20 mbar.

4,7 g 4-Ethoxymethyl-1-methyl-1,2,3,6 -tetrahydro-pyridin und 3,5 g Maleinsäure werden unter Erwärmen in Essigester gelöst. Beim Abkühlen erhält man 4,7 g des Maleinats (Fp. 88-89 ° C).

| $C_9H_{17}NO$ x C $C_4H_4O_4$ (271.32) | | | |
|---|---|---|---|
| ber.<br>gef. | C 57.55<br>57.71 | H 7.80<br>7.62 | N 5.16<br>5.09 |

Citrat: Fp. 124 - 126 ° C (Acetonitril)
$C_9H_{17}NO$ x $C_6H_8O_7$ (347,37)

Beispiel 3:

1-Ethyl-4-methoxymethyl-1,2,3,6 - tetrahydropyridin

6,5 g (0,053 Mol) 4-Methoxymethyl-pyridin und 9,9 g Ethyljodid werden in 65 ml Acetonitril 1 Stunde unter Rückfluß erhitzt. Nach dem Abdestillieren des Lösungsmittels wird das zurückbleibende Quartärsalz in Methanol aufgenommen und analog Beispiel 1 b) mit Natriumborhydrid reduziert.
Man erhält 7,8 g (95,2 % d. Theorie) der Titelverbindung (Kp. 85 ° C/20 mbar).

Das entsprechende Maleinat hat einen Schmelzpunkt von 85-87 ° C (Essigester).

| $C_9H_{17}NO$ x $C_4H_4O_4$ (271,32) | | | |
|---|---|---|---|
| ber.<br>gef. | C 57.55<br>57.50 | H 7.80<br>7.83 | N 5.16<br>5.31 |

Beispiel 4:

4-[(Ethylthio)methyl]-1-methyl-1,2,3,6-tetrahydropyridin

8,2 g (0,054 Mol) 4-[(Ethylthio)methyl]pyridin werden in 60 ml Acetonitril gelöst und dann 9,1 g Methyljodid hinzugefügt. Nach zwölf Stunden wird die Reaktionsmischung im Vakuum eingeengt. Der Rückstand wird analog Beispiel 1 b) mit Natriumborhydrid in Methanol umgesetzt und aufgearbeitet. Das erhaltene Rohprodukt wird destilliert (5,2 g; 56,3 % d. Theorie; Kp. 118-120 ° C/20 mbar) und anschließend an neutralen Aluminiumoxid mit Chloroform als Eluens chromatographiert. Man erhält 4,4 g 4-[(Ethylthio)-methyl]-1-methyl-1,2,3,6-tetrahydropyridin, das mit einem Äquivalent Maleinsäure in das Maleinat (Fp. 102-105 ° C, aus Essigester) überführt wird.

| $C_9H_{17}NS$ x $C_4H_4O_4$ (237.39) | | | | |
|---|---|---|---|---|
| ber.<br>gef. | C 54 33<br>54.25 | H 7.37<br>7.13 | N 4.87<br>4.95 | S 11.16<br>11.08 |

Beispiel 5:

3-Methoxymethyl-1-methyl-1,2,5,6-tetrahydro-pyridin

17,8 g (0,067 Mol) 3-Methoxymethyl-1-methyl-pyridinium-jodid (Fp. 57-59 °C/Isopropanol) werden analog Beispiel 1 b) mit 6,1 g (0,16 Mol) Natriumborhydrid in Methanol reduziert. Das Rohprodukt wird bei vermindertem Druck destilliert (Kp. 63-73 °C/20 mbar) und anschließend säulenchromatographisch ($Al_2O_3$/Essigester) gereinigt.

$^1H$ NMR ($CDCl_3$; 400 MHz)
δ 5,76 (m,1H, CH=); 3.85 (s,2H,O-$CH_2$); 3.29 (s,3H,$OCH_3$); 2.90 (m,2H,$NCH_2$); 2.49 (m,2H,$NCH_2$); 2.37 (s,3H,$NCH_3$); 2.21 (m,2H,$CH_2$-C=).

Das maleinsauere Salz hat einen Schmelzpunkt von 62-64 °C (Essigester/Ether).

| $C_8H_{15}NO$ x $C_4H_4O_4$ (257.29) | | | |
|---|---|---|---|
| ber.<br>gef. | C 56 02<br>55.86 | H 7.44<br>7.67 | N 5.44<br>5.57 |

Beispiel 6:

1,1-Dimethyl-4-methoxymethyl-1,2,3,6-tetrahydropyridinium-bromid

6,8 g (0,072 Mol) Methylbromid in 30 ml Acetonitril werden zu einer Lösung von 5,1 g (0,036 Mol) 4-Methoxymethyl-1-methyl-1,2,3,6-tetrahydro-pyridin in 50 ml Acetonitril getropft.

Nach einer Stunde wird das anfallende Methobromid abgesaugt und aus Acetonitril umkristallisiert.

Ausbeute: 4,8 g (56,3 % d. Theorie); Fp. 139-142 °C (Acetonitril).

| $[C_9H_{18}NO]^+Br^-$ (236.17) | | | | |
|---|---|---|---|---|
| ber.<br>gef. | C 45.77<br>45.66 | H 7.68<br>7.55 | N 5.93<br>5.77 | Br 33.84<br>33.72 |

Nach analogen Verfahren werden folgende Verbindungen erhalten:

Beispiel 7:

1,1-Dimethyl-4-[(ethylthio)methyl]-1,2,3,6-tetrahydro-pyridinium-iodid

| Fp. 132-133 °C (Acetonitril). | | | | |
|---|---|---|---|---|
| ber.<br>gef. | C 38.34<br>38.64 | H 6.44<br>6.68 | N 4.47<br>4.48 | S 10.24<br>10.23 |

Beispiel 8:

4-Methoxymethyl-1,2,3,6-tetrahydro-pyridin

Fumarat: Fp. 115-117 °C (Methanol/Ether).

| $C_7H_{13}NO$ x $C_4H_4O_4$ (243.27) | | | |
|---|---|---|---|
| ber.<br>gef. | C 54.31<br>54.15 | H 7.04<br>6.87 | N 5.76<br>5.72 |

$^1H$ NMR ($CD_3OD$; 90 MHz)
δ 6,75 (s,2H,Fu); 5.82 (m,1H,CH=); 3.87 (m,2H, $OCH_2$); 3.72 (m,2H,$NCH_2$); 3.32 (m,2H,$NCH_2$); 3.31 (s,3H,$OCH_3$); 2.30 (m,2H,$CH_2$-C=)

Beispiel 9:

1-Methyl-4-propoxymethyl-1,2,3,6-tetrahydro-pyridin

Kp. 103-104 °C/20 mbar; Maleinat: Fp. 89-91 °C (Essigester/Ether).

| $C_{10}H_{19}NO$ x $C_4H_4O_4$ (285.35) | | | |
|---|---|---|---|
| ber.<br>gef. | C 58.93<br>58.98 | H 8.12<br>8.10 | N 4.91<br>4.89 |

Beispiel 10:

4-Allyloxymethyl-1-methyl-1,2,3,6-tetrahydro-pyridin

Kp. 103 °C/30 mbar; Fumarat: Fp. 86-88 °C (Acetonitril).

| $C_{10}H_{17}NO$ x 1,5 $C_4H_4O_4$ (341.37) | | | |
|---|---|---|---|
| ber.<br>gef. | C 56.30<br>56.03 | H 6.79<br>6.86 | N 4.10<br>4.28 |

Beispiel 11:

4-Isopropoxymethyl-1-methyl-1,2,3,6-tetrahydro-pyridin

Kp. 90-91 °C/20 mbar; Maleinat; Fp. 108-109 °C (Essigester).

| $C_{10}H_{19}NO$ x $C_4H_4O_4$ (285.35) | | | |
|---|---|---|---|
| ber.<br>gef. | C 58.93<br>58.76 | H 8.12<br>8.09 | N 4.91<br>4.90 |

Beispiel 12:

4-[1-(Methoxy)ethyl]-1-methyl-1,2,3,6-tetrahydro-pyridin

Kp. 76-78 °C/30 mbar; Fumarat: Fp. 94-96 °C (Aceton).

| $C_9H_{17}NO$ x $C_4H_4O_4$ (271.32) | | | |
|---|---|---|---|
| ber.<br>gef. | C 57.55<br>57.62 | H 7.80<br>7.94 | N 5.16<br>5.10 |

Beispiel 13:

1-Methyl-4-[(methylthio)methyl]-1,2,3,6-tetrahydro-pyridin

Kp. 105-106 °C/20 mbar; Maleinat: Fp. 82-85 °C (Essigester).
$^1H$ NMR ($CD_3OD$; 400 MHz)
δ 5.28 (s,2H,Ma); 5.65 (m,1H,CH =); 3.82 (m,2H,$NCH_2$); 3.43 (m,2H,$NCH_2$); 3.10 (s,2H,$SCH_2$); 2.96 (s,3H,N-$CH_3$); 2.58 (m,2H,$CH_2$-C =); 2.00 (s,3H,$SCH_3$).

Beispiele 14:

1-Methyl-4-[(propylthio)methyl]-1,2,3,6-tetrahydro-pyridin

Kp. 127-128 ° C/20 mbar; Oxalat: Fp. 152-154 ° C (Acetonitril).

| $C_{10}H_{19}NS$ x $C_2H_2O_4$ (275.37) | | | | |
|---|---|---|---|---|
| ber. | C 52.34 | H 7.69 | N 5.09 | S 11.64 |
| gef. | 52.82 | 7.87 | 5.21 | 11.65 |

Beispiel 15:

1-Ethyl-4-[(ethylthio)methyl]-1,2,3,6-tetrahydro-pyridin

Kp. 125-126 ° C/30 mbar; Oxalat: 132-134 ° C (Acetonitril).

| $C_{10}H_{19}NS$ x $C_2H_2O_4$ (275.37) | | | | |
|---|---|---|---|---|
| ber. | C 52.34 | H 7.69 | N 5.09 | S 11.64 |
| gef. | 52.61 | 7.89 | 5.24 | 11.55 |

Beispiel 16:

3-[(Ethylthio)methyl]-1-methyl-1,2,5,6-tetrahydro-pyridin-oxalat

Fp. 116-117 ° C (Methanol/Ether).

| $C_9H_{17}NS$ x $C_2H_2O_4$ (261.35) | | | | |
|---|---|---|---|---|
| ber. | C 50.55 | H 7.33 | N 5.36 | S 12.27 |
| gef. | 50.34 | 7.33 | 5.58 | 12.21 |

Beispiel 17:

1-Methyl-4-[2(methylthio)ethyl]-1,2,3,6-tetrahydro-pyridin

Kp. 121-122 ° C/20 mbar; Oxalat: Fp. 110-112 ° C (Acetonitril).

| $C_9H_{17}NS$ x $C_2H_2O_4$ (261.35) | | | | |
|---|---|---|---|---|
| ber. | C 50.55 | H 7.33 | N 5.36 | S 12.27 |
| gef. | 50.46 | 7.48 | 5.45 | 12.31 |

Beispiel 18:

1-Methyl-4-[1(methylthio)ethyl]-1,2,3,6-tetrahydro-pyridin

Kp. 104-106 ° C/20 mbar; Oxalat: Fp. 134-136 ° C (Acetonitril).

| $C_9H_{17}NS$ x $C_2H_2O_4$ (261.35) | | | | |
|---|---|---|---|---|
| ber. | C 50.55 | H 7.33 | N 5.36 | S 12.27 |
| gef. | 50.74 | 7.46 | 5.50 | 12.19 |

Beispiel 19:

3-[(Methylthio)methyl]-1-methyl-1,2,5,6-tetrahydro-pyridin-oxalat

Fp. 119-121 ° C (Acetonitril/Essigester).

| $C_8H_{15}NS$ x $C_2H_2O_4$ (247.32) | | | | |
|---|---|---|---|---|
| ber. | C 48.56 | H 6.93 | N 5.66 | S 12.97 |
| gef. | 48.45 | 6.97 | 5.64 | 13.03 |

Darstellung von Ausgangsverbindungen

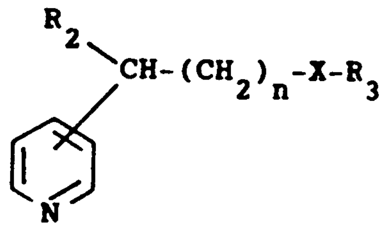

I. 4-(1(Methoxy)ethyl]-pyridin

Eine Lösung von 8,4 g (0,068 Mol) 4-(1-Hydroxyethyl)pyridin in 20 ml absolutem Tetrahydrofuran wird zu einer Suspension von 2 g (0,083 Mol) Natriumhydrid (3,3 g 60 %ige Öldispension; gewaschen in Toluol) in 80 ml absolutem Tetrahydrofuran getropft. Nach beendeter Wasserstoffentwicklung fügt man bei 18-20 ° C 23 g (0,16 Mol) Methyljodid hinzu und lässt zwei Stunden reagieren. Das Reaktionsgemisch wird eingedampft, der Rückstand mit 40 %iger wässeriger Kaliumcarbonat-Lösung versetzt und mit Ether extrahiert. Die organische Phase wird abgetrennt, über Natriumsulfat getrocknet und eingedampft. Nach dem Destillieren des Rohproduktes bei 82-85 ° C/20 mbar erhält man 5,2 g (55,6 % d. Theorie) der Titelverbindung.

II. 4-Isopropoxymethyl-pyridin

3,4 g Natrium werden in 120 ml Isopropanol gelöst und 11 g (0,067 Mol) 4-Chlormethyl-pyridin-hydrochlorid hinzugefügt. Die Reaktionsmischung wird unter Stickstoff und Rühren zehn Stunden zum Rückfluß erhitzt, nach dem Abkühlen mit verdünnter Salzsäure schwach angesäuert und unter vermindertem Druck eingedampft.

Der Rückstand wird mit 40 %iger Kaliumcarbonat-Lösung alkalisch gestellt und mit Ether extrahiert. Nach dem Trocknen mit wasserfreiem Natriumsulfat wird die organische Phase eingedampft. Das Rohprodukt wird anschließend destilliert.

Ausbeute: 5 g (49,3 % d. Theorie); Kp. 95-97 ° C/20 mbar.

III. 4-[(Ethylthio)methyl]-pyridin

Eine Lösung von 19 g (0,28 Mol) Natriumethylat in 200 ml absolutem Ethanol wird mit 8,7 g (0,14 Mol) Ethylmercaptan versetzt und 15 Minuten bei Raumtemperatur gerührt. Anschließend werden 23 g (0,14 Mol) 4-Chlormethyl-pyridin-hydrochlorid hinzugefügt. Nach einer Reaktionszeit von drei Stunden wird die Mischung mit 2N Salzsäure schwach angesäuert, das Lösungsmittel abdestilliert, der Rückstand mit 40 %iger Kaliumcarbonat-Lösung alkalisch gestellt und mit Ether extrahiert. Die organische Phase wird über wasserfreiem Natriumsulfat getrocknet und eingedampft. Bei der Vakuumdestillation des Rückstandes erhält man 17,4 g (81 % d. Theorie) 4-[(Ethylthio)methyl]-pyridin.

Kp. 122-123 ° C/20 mbar.

IV. 4-[2(methylthio)ethyl]-pyridin

Zu einer Lösung von 13,6 g (0,2 Mol) Natriumethylat in 150 ml absolutem Ethanol gibt man 23 g (0,165 Mol) 4-Mercaptoethylpyridin [Lit. L. Bauer und L. A. Gardella, Jr, J. Org. Chem. 26, 82 (1961)] und tropft nach 15 Minuten bei 15-18 ° C 28,1 g (0,2 Mol) Methyljodid zu.

Die Reaktionsmischung wird 30 Minuten bei Raumtemperatur gerührt, mit 2N Salzsäure schwach sauergestellt und im Vakuum eingedampft. Der Rückstand wird mit 40 %iger Kaliumcarbonat-Lösung alkalisch gestellt und mit Ether extrahiert. Nach dem Trocknen über wasserfreiem Natriumsulfat wird die organische Phase eingeengt und das zurückbleibende Öl im Vakuum destilliert.

Kp. 133-134 ° C/20 mbar.

V. 4-[1((Methylthio)ethyl]-pyridin

erhält man analog Beispiel III aus 4-(1-Chlorethyl)-pyridin (Lit. DE-OS 3 334 937 A 1) und Natriumthiomethylat.

Kp. 113-116 ° C/20 mbar.

**Tabellarische Zusammenstellung der Beispiele**

**Tab. 1:**

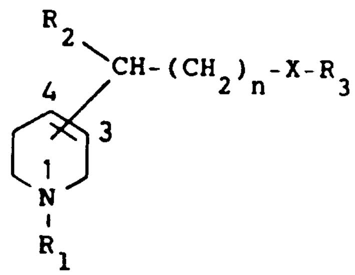

I.

| Beisp. | $R_1$ | $R_2$ | $R_3$ | n | Subst. Pos. | X |
|---|---|---|---|---|---|---|
| 1 | $-CH_3$ | -H | $-CH_3$ | 0 | 4 | O |
| 2 | $-CH_3$ | -H | $-C_2H_5$ | 0 | 4 | O |
| 3 | $-C_2H_5$ | -H | $-CH_3$ | 0 | 4 | O |
| 4 | $-CH_3$ | -H | $-C_2H_5$ | 0 | 4 | S |
| 5 | $-CH_3$ | -H | $-CH_3$ | 0 | 3 | O |
| 8 | -H | -H | $-CH_3$ | 0 | 4 | O |
| 9 | $-CH_3$ | -H | $-n-C_3H_7$ | 0 | 4 | O |
| 10 | $-CH_3$ | -H | $-CH_2-CH=CH_2$ | O | 4 | O |
| 11 | $-CH_3$ | -H | $-CH(CH_3)_2$ | 0 | 4 | O |
| 12 | $-CH_3$ | $-CH_3$ | $-CH_3$ | 0 | 4 | O |
| 13 | $-CH_3$ | -H | $-CH_3$ | 0 | 4 | S |
| 14 | $-CH_3$ | -H | $-n-C_3H_7$ | 0 | 4 | S |
| 15 | $-C_2H_5$ | -H | $-C_2H_5$ | 0 | 4 | S |
| 16 | $-CH_3$ | -H | $-C_2H_5$ | 0 | 3 | S |
| 17 | $-CH_3$ | -H | $-CH_3$ | 1 | 4 | S |
| 18 | $-CH_3$ | $-CH_3$ | $-CH_3$ | 0 | 4 | S |
| 19 | $-CH_3$ | -H | $-CH_3$ | 0 | 3 | S |

**Tab. 2:**

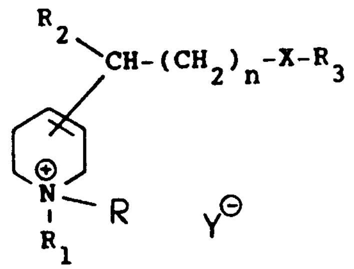

| Beispiel | $R_1$ | $R_2$ | $R_3$ | R | Y | n | Subst. Pos. | X |
|---|---|---|---|---|---|---|---|---|
| 6 | $-CH_3$ | -H | $-CH_3$ | $-CH_3$ | -Br | 0 | 4 | O |
| 7 | $-CH_3$ | -H | $-C_2H_5$ | $-CH_3$ | -I | 0 | 4 | S |

## Patentansprüche

1. Neue Tetrahydropyridin - Derivate der allgemeinen Formel

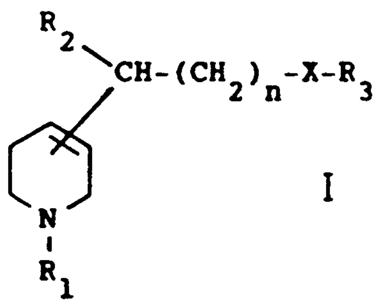

I

worin

$R_1$ Wasserstoff eine verzweigte oder unverzweigte Alkylgruppe mit 1 bis 3 Kohlenstoffatomen;
$R_2$ Wasserstoff oder eine Methylgruppe;
$R_3$ eine verzweigte oder unverzweigte Alkylgruppe mit 1 bis 4 Kohlenstoffatomen, eine Allylgruppe oder eine Propargylgruppe;
X Sauerstoff oder Schwefel und
n eine der Zahlen 0 oder 1 bedeuten, gegebenenfalls als Racemate oder als optisch aktive Verbindungen sowie gegebenenfalls deren Säureadditionssalze wie auch deren quartäre Verbindungen, wobei das 4-(Methoxyethyl)-1-methyl-1,2,3,6-tetrahydropyridinausgeschlossen ist.

2. Neue Tetrahydropyridin-Derivate der allgemeinen Formel I nach Anspruch 1, worin $R_1$ Methyl, $R_2$ Wasserstoff, $R_3$ Methyl, Ethyl oder n-Propyl und n = 0 bedeuten.

3. Neue Tetrahydropyridin-Derivate der allgemeinen Formel I nach Anspruch 1 oder 2, worin sich der Substituent in der 4-Position befindet.

4. Quartäre Tetrahydropyridin-Derivate der allgemeinen Formel

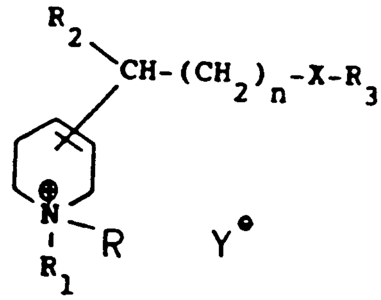

worin $R_1$, $R_2$, $R_3$ und X wie in einem der Ansprüche 1, 2 oder 3 definiert sind und der Ringstickstoff eine Methyl- oder Ethylgruppe als weiteren Substituenten R trägt und Y ein Anion bedeutet.

**5.** Verfahren zur Herstellung von Tetrahydropyridin -Derivaten der allgemeinen Formel I

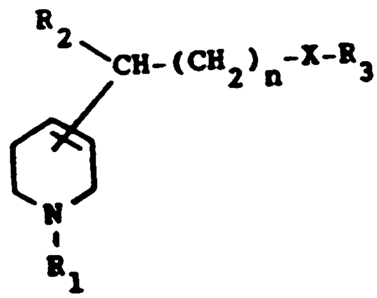

worin

- $R_1$ Wasserstoff, eine verzweigte oder unverzweigte Alkylgruppe mit 1 bis 3 Kohlenstoffatomen;
- $R_2$ Wasserstoff oder eine Methylgruppe;
- $R_3$ eine verzweigte oder unverzweigte Alkylgruppe mit 1 bis 4 Kohlenstoffatomen, eine Allylgruppe oder eine Propargylgruppe;
- X Sauerstoff oder Schwefel und
- n eine der Zahlen 0 oder 1 bedeuten, deren Racemate oder als optisch aktive Verbindungen sowie deren Säureadditionssalze wie auch deren quartäre Verbindungen

dadurch gekennzeichnet, daß man ein Pyridin-Derivat der allgemeinen Formel

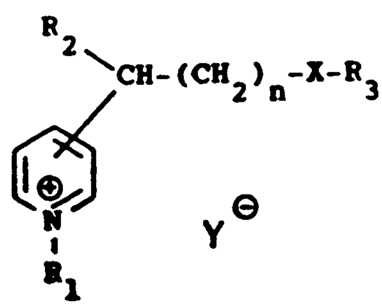

worin $R_1$, $R_2$, $R_3$, X und n die vorgenannte Bedeutung aufweisen mit einem komplexen Metallhydrid reduziert,
anschließend gegebenenfalls zur Herstellung einer Verbindung mit $R_1$ Wasserstoff eine Verbindung der allgemeinen Formel I mit $R_1$ Methyl entmethyliert;
anschließend gegebenenfalls in ihre optisch aktiven Verbindungen auftrennt,
und/oder gegebenenfalls in ihre pharmakologisch unbedenklichen Säureadditionssalze oder ihre quarternären Salze überführt.

6. Pharmazeutische Zubereitung enthalten eine Verbindung der allgemeinen Formel I gemäß Anspruch 1, 2, 3, 4 oder 5 sowie übliche pharmakologisch verträgliche Hilfs- und Trägerstoffe.

7. Verwendung einer Verbindung der allgemeinen Formel I gemäß Anspruch 1, 2, 3, 4 oder 5 zur Herstellung eines Arzneimittels mit cholinomimetischer Wirkung.

**Claims**

1. New tetrahydropyridine derivatives of general formula

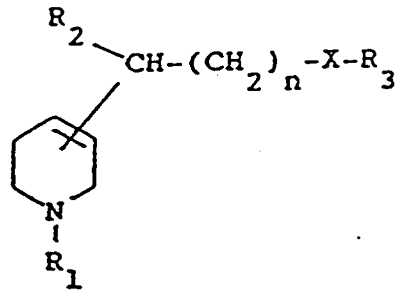

I

wherein

$R_1$ represents hydrogen, a branched or unbranched alkyl group with 1 to 3 carbon atoms;
$R_2$ represents hydrogen or a methyl group;
$R_3$ represents a branched or unbranched alkyl group with 1 to 4 carbon atoms, an allyl group or a propargyl group;
X represents oxygen or sulphur; and
n represents one of the numbers 0 and 1, optionally as racemates or as optically active compounds and optionally the acid addition salts and quaternary compounds thereof, in which the 4-(methylethyl)-1-methyl-1,2,3,6-tetrahyaropyridine is excluded.

2. New tetrahydropyridine derivatives of general formula I as claimed in claim 1, wherein $R_1$ represents methyl, $R_2$ represents hydrogen, $R_3$ represents methyl, ethyl or n-propyl and $n = 0$.

3. New tetrahydropyridine derivatives of general formula I as claimed in claim 1 or 2, wherein the substituent is in the 4-position.

4. Quaternary tetrahydropyridine derivatives of general formula I as claimed in claim 1, 2 or 3,

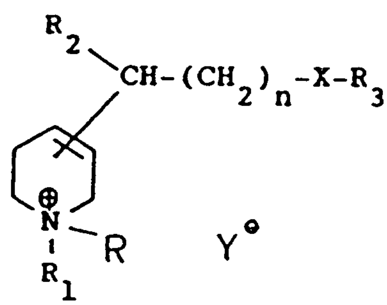

wherein the cyclic nitrogen carries a methyl or ethyl group as further substituent R and Y represents an anion.

5. Process for preparing tetrahydropyridine derivatives of general formula I

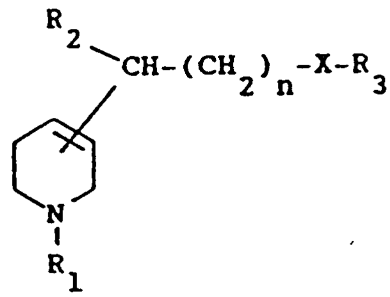

wherein

- $R_1$ represents hydrogen, a branched or unbranched alkyl group with 1 to 3 carbon atoms;
- $R_2$ represents hydrogen or a methyl group;
- $R_3$ represents a branched or unbranched alkyl group with 1 to 4 carbon atoms, an allyl group or a propargyl group;
- X represents oxygen or sulphur; and
- n represents one of the numbers 0 and 1, optionally as racemates or as optically active compounds and optionally the acid addition salts and quaternary compounds thereof,

characterised in that a pyridine derivative of general formula

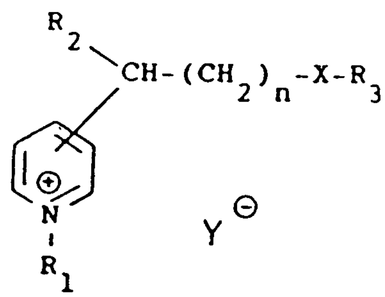

wherein $R_1$, $R_2$, $R_3$, X and n are defined as hereinbefore, is reduced with a complex metal hydride, and subsequently, if desired, in order to prepare a compound wherein $R_1$ represents hydrogen, a compound of general formula I wherein $R_1$ represents methyl is demethylated;
and subsequently, if desired, it is resolved into the optically active compounds thereof,
and/or optionally converted into the pharmacologically harmless acid addition salts or the quaternary salts thereof.

**6.** Pharmaceutical preparation containing a compound of general formula I as claimed in claim 1, 2, 3, 4 or 5 as well as conventional pharmacologically acceptable excipients and carriers.

**7.** Use of a compound of general formula I as claimed in claim 1, 2, 3, 4 or 5 for preparing a pharmaceutical composition with a cholinomimetic activity.

**Revendications**

**1.** Nouveaux dérivés de la tétrahydropyridine de formule générale

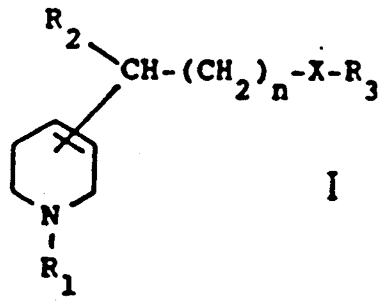

I

dans laquelle
$R_1$ représente l'hydrogène, un groupe alkyle ramifié ou non ramifié de 1 à 3 atomes de carbone;
$R_2$ représente l'hydrogène ou un groupe méthyle;
$R_3$ représente un groupe alkyle ramifié ou non ramifié de 1 à 4 atomes de carbone, un groupe allyle ou un groupe propargyle;
X représente l'oxygène ou le soufre et
n représente l'un des nombres 0 ou 1,
éventuellement sous forme de racémates ou de composés optiquement actifs ainsi éventuellement que leurs sels d'addition d'acide et leurs composés quaternaires, à l'exclusion de la 4-(méthoxyéthyl)-1-méthyl-1,2,3,6-tétrahydropyridine.

2. Nouveaux dérivés de la tétrahydropyridine de formule générale I selon la revendication 1, dans lesquels $R_1$ représente un groupe méthyle, $R_2$ représente l'hydrogène, $R_3$ représente un groupe méthyle, éthyle ou n-propyle et n = 0.

3. Nouveaux dérivés de la tétrahydropyridine de formule générale I selon la revendication 1 ou 2, dans lesquels le substituant se trouve en position 4.

4. Dérivés quaternaires de la tétrahydropyridine de formule générale

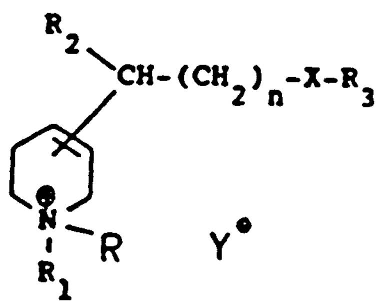

dans laquelle $R_1$, $R_2$, $R_3$ et X sont définis comme dans l'une des revendications 1, 2 ou 3 et l'azote du cycle porte un groupe méthyle ou éthyle comme substituant supplémentaire R et Y représente un anion.

5. Procédé de préparation de dérivés de la tétrahydropyridine de formule générale I

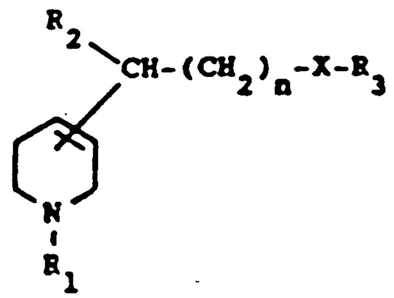

dans laquelle
$R_1$ représente l'hydrogène, un groupe alkyle ramifié ou non ramifié de 1 à 3 atomes de carbone;
$R_2$ représente l'hydrogène ou un groupe méthyle;
$R_3$ représente un groupe alkyle ramifié ou non ramifié de 1 à 4 atomes de carbone, un groupe allyle ou un groupe propargyle;
X représente l'oxygène ou le soufre et
n représente l'un des nombres 0 ou 1,
de leurs racémates ou sous forme de composés optiquement actifs ainsi que de leurs sels d'addition d'acide et de leurs composés quaternaires
caractérisé en ce que l'on réduit avec un hydrure métallique complexe un dérivé de la pyridine de formule générale

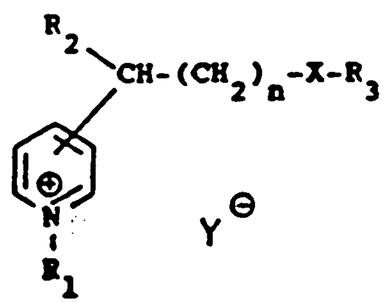

dans laquelle $R_1$, $R_2$, $R_3$, X et n présentent la définition citée précédemment,
puis, éventuellement, pour la préparation d'un composé dans lequel $R_1$ représente l'hydrogène, on déméthyle un composé de formule générale I dans lequel $R_1$ représente un groupe méthyle;
puis on le dédouble éventuellement en ses composés optiquement actifs,
et/ou on le convertit éventuellement en ses sels d'addition d'acide ou en ses sels quaternaires acceptables du point de vue pharmacologique.

6. Préparation pharmaceutique contenant un composé de formule générale I selon la revendication 1, 2, 3, 4 ou 5 ainsi que des adjuvants et véhicules acceptables du point de vue pharmacologique habituels.

7. Utilisation d'un composé de formule générale I selon la revendication 1, 2, 3, 4 ou 5 pour la préparation d'un médicament à effet cholinomimétique.